# EUROPEAN PATENT APPLICATION

(11) **EP 4 386 769 A1**
(43) Date of publication of application: **19.06.2024**
(21) Application number: 22212813.4
(22) Date of filing: 12.12.2022
(51) Int. Cl.: G16H 20/00, G06F 16/14, G06F 16/24, G06F 16/2457, G06F 16/38, G06F 16/432, G06F 16/435, G16H 50/20, G16H 50/70

(54) **GENERATING DATA RESPONSIVE TO A SEARCH ENQUIRY**

(71) Applicant: Koninklijke Philips N.V., 5656 AG Eindhoven (NL)
(72) Inventor: THOMAS PATTARMATAM, Saju, Eindhoven (NL); MALLA, Eswar, Eindhoven (NL); PUTHUKUDI, Swaroop, Eindhoven (NL)
(74) Representative: Philips Intellectual Property & Standards

(57) **Abstract**

A mechanism for processing a search enquiry, for one or more signs/symptoms, provided by a user. A machine-learning method processes the search enquiry together with sensor data of the user produced by one or more health/care devices. The machine-learning method outputs search results for the search enquiry, health information for the user and/or a health recommendation for the user.

## Description

### FIELD OF THE INVENTION

The present invention relates to the field of search engines, particularly to the processing of one or more search enquiries.

### BACKGROUND OF THE INVENTION

The use of search engines or searching tools is becoming increasingly ubiquitous. Typically, a search engine will receive a search inquiry from a user (e.g., via a user interface) and return one or more search results responsive to the search enquiry. Thus, a search engine will process a search inquiry to produce output data, such as search results.

There is a desire to improve the relevance of search results for a particular user or individual, e.g. to contextualize or personalize the results to the individual.

One existing approach to improve the relevance of search results is to make use of search histories (i.e., previous search enquiries) of the individual. For example, this has been put forward by Speretta, Mirco, and Susan Gauch. "Personalized search based on user search histories." The 2005 IEEE/WIC/ACM International Conference on Web Intelligence (WI'05). IEEE, 2005.

There is an ongoing desire to further and/or otherwise improve the personalization and/or accuracy of processing a search enquiry to improve the relevance of the output data to the user providing the search enquiry.

### SUMMARY OF THE INVENTION

The invention is defined by the claims.

According to examples in accordance with an aspect of the invention, there is provided a processing system for generating data responsive to a search enquiry. The data processor is configured to: receive a search enquiry from a user relating to one or more medical signs or symptoms experienced by the user; access sensor data obtained by one or more personal health and/or personal care devices of the user; and process at least the search enquiry and the sensor data using a machine-learning method to produce search results for the search enquiry, health information for the user and/or a health recommendation for the user.

The proposed system makes use of a search enquiry and sensor data in order to generate a contextually relevant response to the search enquiry or a recommendation responsive to the search enquiry.

This proposed approach recognizes that there is a causal connection or link between a search made by a user (regarding their medical sign(s)/symptom(s)) and previous use of a personal health and/or care device. As a basic example, there is an increased likelihood that a user that has recently used a hair removal device would search for "skin irritation". This recognition could be exploited to provide health information, health recommendations or search results that make use of the contextual history of health/personal care device usage.

The proposed technique can be used to provide the user with improved healthcare information to more readily understand the state of their health or their body. In particular, the proposed technique can facilitate improved determination of search results (or healthcare information/recommendations) that are specific to the user's environment and/or scenario.

The data processor may be configured to access the sensor data from a database in which sensor data is stored in respect of a plurality of personal health and/or personal care devices for a plurality of users. Thus, there may be a shared database, for example maintained by the manufacturer of a suite of personal health devices. This approach improves an efficiency of obtaining or receiving the sensor data.

The one or more personal health and/or personal care devices may comprise one or more of: a shaver; an electric toothbrush; a hair removal device; and a sleep monitoring device. These are examples of possible personal health device that may have sensing functionality. The sensing data may at least indicates times when the device has been used (hence time periods of use), and preferably also other parameters such as device settings and/or health data.

The data processor may be further configured to access one or more previous search enquiries of the user; and process at least the search enquiry, the one or more previous search enquiries and the sensor data using the machine-learning method to produce the search results for the search enquiry, the health information for the user and/or the health recommendation for the user.

The machine-learning method may be configured to: receive, as input data, at least the search enquiry and the sensor data; and provide, as output data, the search results for the search enquiry, health information for the user and/or a health recommendation for the user.

The sensor data preferably comprises at least usage information of the one or more personal health and/or personal care devices by the user.

Preferably, the usage information comprises time data indicating any times at which the one or more personal health and/or personal care devices by the user have been used by the user.

In some examples, the personal care device is a health monitoring device and the sensor data comprises at least health monitoring information by the health monitoring device.

The health monitoring device may, for instance, be a sleep monitoring device and the sensor data may correspondingly comprise sleep monitoring information by the sleep monitoring device.

The data processor may be configured to control a display to present the search results for the search enquiry, health information for the user and/or a health recommendation for the user. This provides a user-friendly way to provide the data produced by the machine-learning method to the user.

The data processor may be configured to control the display to present the search results for the search enquiry, health information for the user and/or a health recommendation for the user in association with a 3D human body model. This enables the user to have a better understanding of the data produced by the machine-learning method.

There is also provided a computer-implemented method for generating data responsive to a search enquiry. The computer-implemented method comprises: receiving a search enquiry from a user relating to one or more medical signs or symptoms experienced by the user; accessing sensor data obtained by one or more personal health and/or personal care devices of the user; and processing at least the search enquiry and the sensor data using a machine-learning method to produce search results for the search enquiry, health information for the user and/or a health recommendation for the user.

The method may further comprise accessing one or more previous search enquiries of the user, wherein the step of processing at least the search enquiry and the sensor data comprises processing at least the search enquiry, the one or more previous search enquiries and the sensor data using the machine-learning method to produce the search results for the search enquiry, the health information for the user and/or the health recommendation for the user.

Preferably, the machine-learning method is configured to: receive, as input data, at least the search enquiry and the sensor data; and provide, as output data, the search results for the search enquiry, health information for the user and/or a health recommendation for the user.

There is also proposed a computer program comprising computer program code means which is adapted, when said program is run on a computer, to implement any herein described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which embodiments can be employed;
Fig. 2 illustrates a proposed processing system; and
Fig. 3 is a flow-chart illustrating a proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for processing a search enquiry, for one or more signs/symptoms, provided by a user. A machine-learning method processes the search enquiry together with sensor data of the user produced by one or more health/care devices. The machine-learning method outputs search results for the search enquiry, health information for the user and/or a health recommendation for the user.

Embodiments are based on the realization that sensor data can be combined with a search enquiry to improve the personalization of a search engine and/or provide health recommendations/information to an individual, as the search enquiry will include information on signs/symptoms that may be associated with the use of such devices. This means that more accurate identification of a potential cause of the signs/symptoms in the search enquiry can be identified and used to contextualize or improve the results.

Approaches may be employed in any scenario or environment in which a user may wish to provide a search enquiry. For instance, approaches can be integrated into mobile device applications that include a search function or tool, particularly mobile health applications.

In the context of the present invention, a user is an individual or person that uses one or more personal health devices or personal care devices (that produce sensor data) and is able to provide a search enquiry, e.g., via a user interface

The present disclosure makes use of a machine-learning method to process a search enquiry together with additional information in order to provide output data such as search results. Thus, the machine-learning method may be able to effectively act as a search engine or other search enquiry processing technique.

Techniques that make use of machine-learning methods in order to perform the function of a search engine are widely known. Examples include: McCallum, Andrew, et al. "A machine learning approach to building domain-specific search engines." IJCAI. Vol. 99. 1999; Karwa, Rushikesh, and Vikas Honmane. "Building search engine using machine learning technique." 2019 International Conference on Intelligent Computing and Control Systems (ICCS). IEEE, 2019; Neogy, Taposh Kumar, and Harish Paruchuri. "Machine Learning as a New Search Engine Interface: An Overview." Engineering International 2.2 (2014): 103-112; and Beitzel, Steven M., et al. "Improving automatic query classification via semi-supervised learning." Fifth IEEE International Conference on Data Mining (ICDM'05). IEEE, 2005. Other approaches will be readily apparent to the skilled person familiar with the processing of search enquiries.

The present invention proposes to supplement or improve any such known machine-learning methods by making further use of sensor data (produced by one or more personal health/care devices) as input to the machine-learning method. The sensor data thereby acts as an additional input feature (i.e., in additional to the search enquiry) for a machine-learning method to produce output data.

The skilled person would readily appreciate, upon being taught that sensor data can be used to improve the processing of a search enquiry (e.g., to provide bespoke/specific results/information/advice) that there are a wide variety of possible data outcomes that are dependent upon the content of the search enquiry and/or the function/purpose of the devices that provide the sensor data. The underlying concept relates to the blending of search enquiries and sensor data.

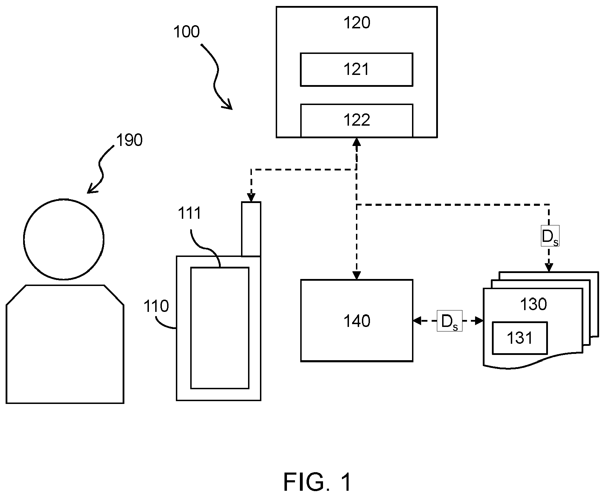

Fig. 1 illustrates a system 100 in which embodiments of the invention can be employed.

The system 100 comprises a user interface 110, a processing system 120 and one or more personal health/care devices 130 for a user 190. The processing system 120, by itself, provides an example embodiment of the proposed invention.

The user interface 110 is configured to allow or permit a user 190 to provide or input data to the processing system 120. Thus, the user interface 110 is communicatively coupled to the processing system 120.

The processing system 120 comprises a data processor 121 configured to process data, e.g., at least data received from the user interface. The processing system may be integrated into a same device as the user interface (e.g., a processing system of a mobile/cellular device) or may be at a separate location to the user interface (e.g., a cloud-computing system).

Each personal health/care device 130 comprises a sensor 131 for producing sensor data of the user. Thus, in practice, each personal health/care device is used by the user 190, which generates sensor data responsive to the use of said device. Examples of suitable devices 130 include: a shaver; an electric toothbrush; an electrical oral care device (e.g., an oral irrigator); a nutritional intake monitor; a hair removal device (e.g., an epilator or an IPL hair removal device); and a sleep monitoring device. Oher suitable examples will be readily apparent to the skilled person.

The user interface 110 is configured to permit the user to provide a search enquiry relating to one or more medical signs and/or symptoms experienced by the user. The user interface 110 provides such a search enquiry to the processing system. The search enquiry may be in the form of textual data and/or image data (e.g., an image of a sign/symptom to be searched).

The data processor 121 of the processing system 120 thereby receives the search enquiry from the user (e.g., via an input/output interface 122).

The data processor 121 is also configured to access the sensor data produced or obtained by the one or more personal health and/or personal care devices 130 of the user 190. This can be achieved, for instance, by the data processor 121 directly accessing the sensor data from the device(s) 130. Alternatively, the data processor 121 may extract the sensor data from a database 140 (e.g., a memory or storage unit) that stores data generated by the personal health and/or personal care device(s) 130. Of course, a combination of both of these approaches could be used, e.g., sensor data could be extracted from both the device(s) 130 (directly) and from the database 140.

The database 140 may be configured such that the sensor data (for the user 190) is stored in respect of a plurality of personal health and/or personal care devices for a plurality of users, including the user 190 and at least one other user. The at least one other user may comprise no fewer than 100 other users, e.g., no fewer than 1000 other users.

The sensor data produced by the health/care device(s) 130 preferably includes usage information of the one or more personal health and/or personal care devices by the user.

Preferably, the usage information comprises time data indicating any times at which the one or more personal health and/or personal care devices by the user have been used by the user. It has been recognized that the times at which the device(s) is/are used will influence current signs/symptoms of the user (e.g., recent use of certain devices may result in certain symptoms). By taking the times at which the device(s) were used into account, more accurate generation of health information and/or recommendations can be generated for the use.

Other example data for inclusion in sensor data may include device settings, which will be device dependent (e.g., for a toothbrush this may include: brushing pattern; rotation speed; vibration amplitude and so on). There is some influence on the sign/symptoms of the user responsive to the device settings of the device(s), which could be used to provide useful feedback to the user. For instance, high amplitude vibrations of a toothbrush may result in sensitive teeth, such that a user providing "sensitive teeth" as a search enquiry be provided with advice about the vibration amplitude by the machine-learning method.

As another example, the sensor data may include user monitoring data, i.e., data sensed by the device(s) 130 responsive to a sign/symptom of the user 190. User monitoring data is, of course, dependent upon the purpose of the device 130, for instance, a sleep monitoring device will produce sleep monitoring data responsive to a time/quality/status of the user's sleep. Other suitable examples will be apparent to the skilled person.

Thus, in general, the sensor data comprises information responsive to a user of the health/care device by the user 190, e.g., responsive to at least one interaction between the user 190 and the health/care device.

The data processor 121 is configured to process at least the search enquiry (produced at/by the user interface 110) and the sensor data (produced at/by the personal health and/or personal care device(s) 130) using a machine-learning method to produce: search results for the search enquiry, health information for the user and/or a health recommendation for the user.

Thus, the machine-learning method is configured to: receive, as input data, at least the search enquiry and the sensor data; and provide, as output data, the search results for the search enquiry, health information for the user and/or a health recommendation for the user.

For the purpose of conciseness, the search results for the search enquiry, health information for the user and/or a health recommendation for the user can be collectively labelled "output data".

Search results for the search enquiry may, for instance, provide more contextualized search results for the specific enquiry of the user. The health information may, for instance, provide information relating to the sign(s)/symptom(s) of the user in the context of their usage of the personal health/care device(s), e.g., whether or not the sign(s)/symptom(s) can be attributed to the use of the personal health/care device. The health recommendation may, for instance, provide recommendations for future use of the personal health/care device(s) used by the user responsive to the sign(s)/symptom(s), e.g., reduced usage or specific forms of usage.

The present disclosure thereby provides a mechanism for processing a search enquiry of a user (input at a user interface) that provide contextualized results - i.e., results that take into account activity with the health/care device(s) that have been used by the user. This reduces potential confusion and/or the provision of potentially misleading information to the user 190.

The proposed system also facilitates the blending of information from multiple sources in order to provide contextually useful health information and/or results to the user.

The output of the machine-learning method thereby facilitates the identification of information about the user, e.g., health information and/or health recommendations, that facilitate improved understanding about the state of the user (in the form of health information) and/or guide in the performance of one or more technical tasks that would improve the health of the user (in the form of the health recommendation(s)).

Any search results produced by the proposed method also provide improved understanding of the state of the user, as the results are contextualized to the use of the health/care device(s) used by the user.

The data processor may be configured to control a display to present the search results for the search enquiry, health information for the user and/or a health recommendation for the user. In particular, the data processor 121 may be configured to control a display 111 of the user interface 110 (e.g., via the input/output interface 122) to provide a visual representation of the output of the machine-learning method to the user.

The data processor may be configured to control the display to present the search results for the search enquiry, health information for the user and/or a health recommendation for the user in association with a 3D human body model. This enables the user to have a better understanding of the data produced by the machine-learning method.

Of course, there are other uses for the data produced by the data processor. For instance, the output of the machine-learning method may be stored in a database or memory for later retrieval. As another example, the data produced by the data processor may be further processed or passed to a further processor for further processing.

Thus, a typical workflow that facilitates the use of embodiments of the invention is as follows. Firstly, the user 190 will use one or more personal health/care devices 130. The device(s) 130 produce sensor data D_{S} responsive to the usage of said device(s), which may be stored in the database 140. Subsequently, the user initiates a search for one or more signs and/or symptoms using the user interface 110 (e.g., provides a textual input representative of the one or more signs and/or symptoms), thereby producing a search enquiry. The data processor 121 of the processing system receives the search enquiry and accesses the database 140 to retrieve the sensor data D_{S}. The data processor 121 then processes at least the search enquiry and the sensors data to produce an appropriate result (i.e., search results for the search enquiry, health information for the user and/or a health recommendation for the user).

In some embodiments, the machine-learning method may process additional information in producing the search results for the search enquiry, the health information for the user and/or the health recommendation for the user.

For instance, in at least one example, the data processor 121 is configured to access one or more previous search enquiries of the user. Thus, search enquiries of the user may be stored, e.g., in the database 140, and accessed by the data processor 121.

Accordingly, the data processor 121 may be configured to process at least the search enquiry, the one or more previous search enquiries and the sensor data using the machine-learning method to produce the search results for the search enquiry, the health information for the user and/or the health recommendation for the user.

In some examples, the data processor is configured to process at least the search enquiry, the one or more previous search enquiries and user data using the machine-learning method to produce the search results for the search enquiry, the health information for the user and/or the health recommendation for the user.

The user data may comprise information about the user, e.g., properties or characteristics of the user (e.g., age, gender, medical history and so on). This provides further useful contextualizing information for improving the generation of data by the machine-learning method.

In some examples, the data processor is configured to process at least the search enquiry, the one or more previous search enquiries and time information using the machine-learning method to produce the search results for the search enquiry, the health information for the user and/or the health recommendation for the user.

The time information may, for instance, indicate a current time and/or season. The current time or season has been identified as having an impact on the health condition and health recommendations of an individual, such that the status of the individual may be attributable to the current time/season. For instance, a sign of "dry skin" may be attributable to colder seasons. By taking this information into account, more accurate output data can be generated by the machine-learning method. As an example, a user inputting a symptom of "sinus flare up" in winter could be advised to carry a head cap or shawl (as the sinus flare up can be attributed to the cold weather).

In some examples, the data processor is configured to process at least the search enquiry, the one or more previous search enquiries and weather information using the machine-learning method to produce the search results for the search enquiry, the health information for the user and/or the health recommendation for the user.

The state of the weather will also impact the health condition and/or health recommendations of an individual. For instance, cold weather may increase the prevalence of certain signs/symptoms (e.g., dry skin, sinus flare ups, poor blood circulation) whereas hot weather may increase the prevalence of other signs/symptoms (e.g., sunburn, sensitive skin or the like). By taking this information into account, more accurate output data can be generated by the machine-learning method.

Of course, any of the above-described instances of additional information could be used in combination. Thus, the data processor may be configured to process at least the search enquiry, the one or more previous search enquiries and at least one of the following: any previous search enquiries; user data; time information; and/or weather data.

The present disclosure makes use of a machine-learning method. A machine-learning method is any self-training algorithm that processes input data in order to produce or predict output data. More particularly, a machine-learning method is configured to learn links or relationships between different data elements or data features.

In the context of the present disclosure, the input data comprises a search enquiry and sensor data and the output data comprises search results for the search enquiry, health information for the user and/or one or more health recommendations for the user.

The use of machine-learning methods to process a search enquiry is well known. The present invention proposes to supplement such machine-learning methods with the use of sensor data to improve the performance of the machine-learning method by making the output data more personalized to the circumstances of the specific user.

Suitable machine-learning methods for being employed in the present invention will be apparent to the skilled person. Examples of suitable machine-learning methods include decision tree algorithms and artificial neural networks. Other machine-learning methods such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning method are well known. Typically, such methods comprise obtaining a training dataset, comprising training input data entries and corresponding training output data entries. An initialized machine-learning method is applied to each input data entry to generate predicted output data entries. An error between the predicted output data entries and corresponding training output data entries is used to modify the machine-learning method. This process can repeated until the error converges, and the predicted output data entries are sufficiently similar (e.g. ±1%) to the training output data entries. This is commonly known as a supervised learning technique.

For example, where the machine-learning method is formed from a neural network, (weightings of) the mathematical operation of each neuron may be modified until the error converges. Known methods of modifying a neural network include gradient descent, backpropagation algorithms and so on.

The training input data entries correspond to example search enquiries and corresponding sensor data. The training output data entries correspond to corresponding example search results for the search enquiry, health information for the user and/or one or more health recommendations for the user.

At least some of the training output data entries may, for instance, be produced or selected by an appropriately skilled or experienced clinician. For instance, the clinician may analyze the search enquiry and the sensor data to prepare or produce a training output data entry.

As another example, at least some of the training output data entries may be produced by converting the example sensor data into additional text for the search enquiry (e.g., using a text generation approach), and then using a conventional search engine to produce search results that make use of both the search enquiry and the sensor data. The produced search results may act as the training output data entry.

As yet another example, at least some of the training output data entries may be produced based on past or previous usage data by the user (and/or other users). For instance, the selection of one or more search results by a user with certain sensor data, or health information found in a selected search result, following a search enquiry may be used a training output data entry. The corresponding training input data entry may include the search enquiry and the sensor data associated with the user at the time of processing the search enquiry.

The skilled person would readily appreciate how the training input data entries can be modified or expanded to include other forms of additional information.

The processing system may be configured to produce the output data responsive to receiving a search enquiry, i.e., to provide immediate feedback for the user providing the search enquiry.

In some examples, the processing system is configured to produce the output data periodically (e.g., once an hour, once a week or once a month). This approach can, for instance, facilitate provision of insights or reminders for the user. This approach is particularly useful to respond to changes in the use of the sensor data.

Thus, the processing system is able to effectively act as a notification system, to notify or remind the user about their health status and/or health recommendations.

In some examples, the processing system is configured to produce the output data responsive to a user prompt. Thus, an individual may be able to prompt, e.g., via the user interface, the processing system to process the input data, using the machine-learning method, to produce the output data.

A number of example scenarios for processing of a search enquiry and sensor data to produce suitable output data are hereafter described.

As an example, use of an IPL hair removal device for an individual with folliculitis may result in a rash or reaction. It may be known that the magnitude of this rash reduces with frequent use of the IPL hair removal device. In this scenario, the machine-learning method may receive a search enquiry indicating "rash" as well as sensor data indicating that an IPL hair removal device has been recently use. This knowledge can be used to provide a health recommendation that if the rash followed use of the IPL hair removal device, then the magnitude of the rash will decrease with frequent use. Also for people with folliculitis tendencies, the inflammation starts again when hair starts to grow again due to ingrown hair. When this knowledge is paired with device usage data, it is possible to generate a health reminder for the user to use the device again via a notification/reminder service.

In another example, it may be known that use of a shaver causes a rash if a user has an inflammatory skin disease. It is also known that quality of sleep has an impact on inflammatory skin disease. This knowledge may be used to provide useful output data if an individual searches for "rash", with sensor data indicating that they have recently used a shaver and have had a bad night's sleep (e.g., as detected by a sleep monitoring device). Thus, clinically useful output data can be provided to guide or aid the user into understanding a potential cause of a rash.

As yet another example, sensor data may include food/calorie intake (as provided at a food monitoring device) and brushing information (as monitored at a toothbrush). If an individual searches for "acid reflux", this information can be combined with the sensor data to determine or predict whether or not the user is likely to suffer from tooth decay. Appropriate health information and/or recommendations can be provided responsive to this information.

These example scenarios clearly demonstrate that there are a multitude of causal connections between device data/usage and search enquiries, as well as forms and structures for output data that could be produced using such information. The connections/correlations can be identified by a machine-learning method and used to contextualize information provided by or contained in the output data for increased personalization to the specific user's circumstances. The skilled person would appreciate that these are only a small selection of all possible forms of input and output data for an appropriately trained machine-learning method that all rely upon the same underlying principle of processing a search enquiry and sensor data to produce the output data. Proposed embodiments rely upon this underlying principle in order to provide a wide variety of different forms of output data. The precise mechanism or form of input/output data is immaterial to the underlying inventive principle.

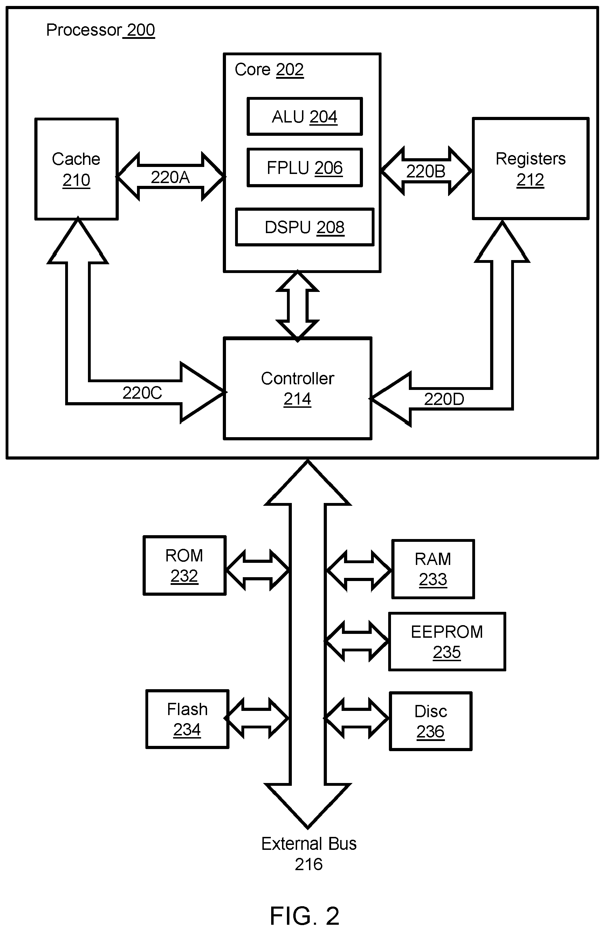

Fig. 2 is a block diagram illustrating an example processing system 200 according to embodiments of the disclosure. The processing system 200 may be used to implement one or more processing system described herein, for example, processing system 120 shown in Fig. 1. Continued reference to the features of Fig. 1 will be hereafter described, where appropriate.

Processing system 200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processing system 200 may include one or more cores 202 (which here act as the data processor). The core 202 may include one or more arithmetic logic units (ALU) 204. In some embodiments, the core 202 may include a floating point logic unit (FPLU) 206 and/or a digital signal processing unit (DSPU) 208 in addition to or instead of the ALU 204.

The processing system 200 may include one or more registers 212 communicatively coupled to the core 202. The registers 212 may be implemented using dedicated logic gate circuits (e.g., flip-flops) and/or any memory technology. In some embodiments the registers 212 may be implemented using static memory. The register may provide data, instructions and addresses to the core 202.

In some embodiments, processing system 200 may include one or more levels of cache memory 210 communicatively coupled to the core 202. The cache memory 210 may provide computer-readable instructions to the core 202 for execution. The cache memory 210 may provide data for processing by the core 202. In some embodiments, the computer-readable instructions may have been provided to the cache memory 210 by a local memory, for example, local memory attached to the external bus 216. The cache memory 210 may be implemented with any suitable cache memory type, for example, metal-oxide semiconductor (MOS) memory such as static random access memory (SRAM), dynamic random access memory (DRAM), and/or any other suitable memory technology.

The processing system 200 may include a controller 214, which may control input to the processing system 200 from other processors and/or components included in a system (e.g., the user interface 110 and/or database 140 from Fig. 1) and/or outputs from the processing system to other processors and/or components included in the system (e.g., the user interface 110). Controller 214 may control the data paths in the ALU 204, FPLU 206 and/or DSPU 208. Controller 214 may be implemented as one or more state machines, data paths and/or dedicated control logic. The gates of controller 214 may be implemented as standalone gates, FPGA, ASIC or any other suitable technology.

The registers 212 and the cache 210 may communicate with controller 214 and core 202 via internal connections 220A, 220B, 220C and 220D. Internal connections may implemented as a bus, multiplexor, crossbar switch, and/or any other suitable connection technology.

Inputs and outputs for the processing system 200 may be provided via a bus 216, which may include one or more conductive lines. The bus 216 may be communicatively coupled to one or more components of processing system 200, for example the controller 214, cache 210, and/or register 212. The bus 216 may be coupled to one or more components of the system, such as components 110, 140 mentioned previously.

The bus 216 may be coupled to one or more external memories. The external memories may include Read Only Memory (ROM) 232. ROM 232 may be a masked ROM, Electronically Programmable Read Only Memory (EPROM) or any other suitable technology. The external memory may include Random Access Memory (RAM) 233. RAM 233 may be a static RAM, battery backed up static RAM, Dynamic RAM (DRAM) or any other suitable technology. The external memory may include Electrically Erasable Programmable Read Only Memory (EEPROM) 235. The external memory may include Flash memory 234. The External memory may include a magnetic storage device such as disc 236. In some embodiments, the external memories may be included in a system, such as the database 140.

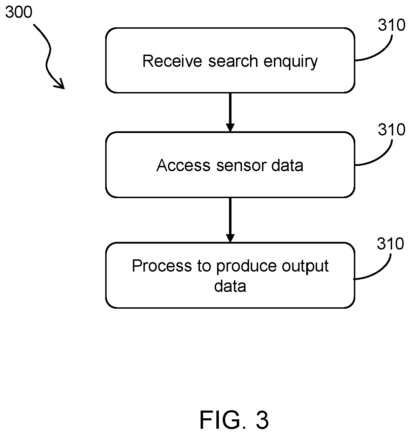

Fig. 3 is a flowchart illustrating a computer-implemented method 300 according to an embodiment.

The computer-implemented method 300 comprises a step 310 of receiving a search enquiry from a user relating to one or more medical signs or symptoms experienced by the user.

The method 300 also comprises a step 320 of accessing sensor data obtained by one or more personal health and/or personal care devices of the user.

The method 300 also comprises a step 330 of processing at least the search enquiry and the sensor data using a machine-learning method to produce search results for the search enquiry, health information for the user and/or a health recommendation for the user.

The skilled person would be readily capable of adapting the method 300 to carry out the steps performing by any previously described processing system, mutatis mutandis.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

### Simulations and results:

Simulations have been performed whereby for each simulation two search queries are performed by the user.

A first search query is performed without any additional sensor data that is obtained by a personal health and/or personal care device of the user. It is determined how many clicks of the user are necessary to arrive at the right cause for a certain health issue.

A second search query is performed including additional sensor data that obtained by a personal health and/or personal care device of the user. It is determined how many clicks of the user are necessary to arrive at the right cause for a certain health issue.

The first simulation data relates to search queries for possible causes of folliculitis.

Simulation data 1:
1. Traditional search:
   a. A user searches for possible causes of folliculitis, gets an array of non-relevant data.
      Thereafter, the user adds "folliculitus after using an intense pulsed light hair removal device", user gets the top result of a community discussion of the same. Thereafter, the user scrolls through data to solution and possible cause.
   b. Number of clicks: 3
2. Improved search by invention:
   a. System already has information about user's past intense pulsed light hair removal device usage, e.g. originating from an pulsed light hair removal device having sensors onboard to fetch required data, and has FAQ data fed in about possible reactions etc. User searches for "possible causes of folliculitis" and he/she is immediately presented with the data.
   b. Number of clicks: 1

The simulation data shows that the number of clicks required by the user to arrive at the right result is reduced from three to one. The reduction of clicks results in a reduction of instructions received by the data processor thereby reducing overall power consumption of the processor.

Further, the invention may make use of a network connection whereby bandwidth usage directly depends on the number of clicks. Each click may require a communication to be established with a server and the user's device that is executing the search query. During each communication, data is transmitted over the network. Less clicks results in a lower use of network bandwidth.

The second simulation data relates to search queries for reason for frequent tooth decay.

Simulation data 2:
1. Traditional search:
   a. A user searches for reasons for frequent tooth decay. A traditional search points to an array of results ranging from lack of brushing habits to low immunity. Thereafter, the user refines search to include use of electric toothbrush regularly. The user still gets result about low immunity, acid reflux etc. Thereafter, the user can get a correct result by further narrowing search to acid reflux.
   b. Number of clicks: 4-5
2. Improved search by invention:
   a. Data is available on the user's tooth brushing routine, e.g. originating from an electric and connected toothbrush having sensors onboard to fetch required data, and can fetch previous meal time from apple health kit or google fit, and can conclude relation to possible acid reflux on the first search attempt.
   b. Number of clicks: 1

The simulation data shows that the number of clicks required by the user to end up at the right result is reduced from 4-5 to 1. The reduction of clicks results in a reduction of instructions received by the data processor thereby reducing overall power consumption of the processor.

Further, the invention may make use of a network connection whereby bandwidth usage directly depends on the number of clicks. Each click may require a communication to be established between a server and the user's device that is executing the search query. During each communication, data is transmitted over the network. Less clicks results in a lower use of network bandwidth.

By the present invention performing the search and arriving at the most accurate search results in a resource efficient manner is achieved by using the sensor data obtained by the personal care devices. Hence the data used to improve search efficiency is not subjective to the choices made by the user but objective and of a technical nature as it is based on the measurements of the sensors used.

## Claims

1. A processing system (120) for generating data responsive to a search enquiry, comprising a data processor (121) configured to:
receive (310) a search enquiry from a user (190) relating to one or more medical signs or symptoms experienced by the user;
access (320) sensor data obtained by one or more personal health and/or personal care devices (130) of the user; and
process (330) at least the search enquiry and the sensor data using a machine-learning method to produce search results for the search enquiry, health information for the user and/or a health recommendation for the user.

2. The processing system of claim 1, wherein the data processor is configured to access the sensor data from a database (140) in which sensor data is stored in respect of a plurality of personal health and/or personal care devices for a plurality of users.

3. The processing system of claim 1 or 2, wherein the one or more personal health and/or personal care devices comprise one or more of:
a shaver;
an electric toothbrush;
a hair removal device; and
a sleep monitoring device.

4. The processing system of any of claims 1 to 3, wherein the data processor is further configured to:
access one or more previous search enquiries of the user; and
process at least the search enquiry, the one or more previous search enquiries and the sensor data using the machine-learning method to produce the search results for the search enquiry, the health information for the user and/or the health recommendation for the user.

5. The processing system of any of claims 1 to 4, wherein the machine-learning method is configured to:
receive, as input data, at least the search enquiry and the sensor data; and
provide, as output data, the search results for the search enquiry, health information for the user and/or a health recommendation for the user.

6. The processing system of any of claims 1 to 5, wherein the sensor data comprises at least usage information of the one or more personal health and/or personal care devices by the user.

7. The processing system of claim 6, wherein the usage information comprises time data indicating any times at which the one or more personal health and/or personal care devices by the user have been used by the user.

8. The processing system of any of claims 1 to 7, wherein the personal care device is a health monitoring device and the sensor data comprises at least health monitoring information by the health monitoring device.

9. The processing system of claim 8, wherein the health monitoring device is a sleep monitoring device and the sensor data comprises sleep monitoring information by the sleep monitoring device.

10. The processing system of any one of claims 1 to 9, wherein the data processor is configured to control a display to present the search results for the search enquiry, health information for the user and/or a health recommendation for the user.

11. The processing system of claim 10, wherein the data processor is configured to control the display to present the search results for the search enquiry, health information for the user and/or a health recommendation for the user in association with a 3D human body model.

12. A computer-implemented method (300) for generating data responsive to a search enquiry, the computer-implemented method comprising:
receiving (310) a search enquiry from a user (190) relating to one or more medical signs or symptoms experienced by the user;
accessing (320) sensor data obtained by one or more personal health and/or personal care devices (110) of the user; and
processing (330) at least the search enquiry and the sensor data using a machine-learning method to produce search results for the search enquiry, health information for the user and/or a health recommendation for the user.

13. The computer-implemented method of claim 12, further comprising accessing one or more previous search enquiries of the user,
wherein the step of processing at least the search enquiry and the sensor data comprises processing at least the search enquiry, the one or more previous search enquiries and the sensor data using the machine-learning method to produce the search results for the search enquiry, the health information for the user and/or the health recommendation for the user.

14. The computer-implemented method of claim 12 or 13 wherein the machine-learning method is configured to:
receive, as input data, at least the search enquiry and the sensor data; and
provide, as output data, the search results for the search enquiry, health information for the user and/or a health recommendation for the user.

15. A computer program comprising computer program code means which is adapted, when said program is run on a computer, to implement the method of any one of claims 12 to 14.
